Europäisches Patentamt

European Patent Office

Office européen des brevets

(11) Publication number: **0 318 184**
**A1**

## (12) EUROPEAN PATENT APPLICATION

(21) Application number: **88310594.2**

(22) Date of filing: **10.11.88**

(51) Int. Cl.4: **A61K 37/36 , A61K 37/24**

(30) Priority: **12.11.87 US 119611**

(43) Date of publication of application:
**31.05.89 Bulletin 89/22**

(84) Designated Contracting States:
**ES GR**

(71) Applicant: **SCHERING CORPORATION**
**2000 Galloping Hill Road**
**Kenilworth New Jersey 07033(US)**

(72) Inventor: **Black, Hugh E.**
**83 Ridge Road**
**Sparta New Jersey 07871(US)**

(74) Representative: **Ritter, Stephen David et al**
**Mathys & Squire 10 Fleet Street**
**London EC4Y 1AY(GB)**

(54) **Acceleration of bone formation with GM-CSF.**

(57) Methods and compositions are described that accelerate bone formation using granulocyte macrophage colony stimulating factors (GM-CSFs). The methods include treatments for osteoporosis and bone fractures. The compositions include GM-CSFs in combination with bone-formation stimulators and/or bone-resorption inhibitors.

EP 0 318 184 A1

## ACCELERATION OF BONE FORMATION WITH GM-CSF

### BACKGROUND OF THE INVENTION

This invention relates to the acceleration of bone formation with GM-CSF.

One aspect of this invention relates to treating the degenerative bone disease known as osteoporosis. Osteoporosis is responsible for at least 1.2 million fractures in the United States each year; it is responsible for more than 25,000 deaths annually; and it is estimated to have direct and indirect costs each year of $6.1 billion in the United States alone [Melton et al., The Osteoporotic Syndrome, Grune and Stratton, New York: 45-72 (1983); Cummings et al., Epidemiol. Rev. 7:178 (1985); Riggs et al., New Eng. J. Med. 314:1676 (1986)]. The disease is characterized by osteopenia and fractures.

The underlying problem, separate from the osteopenia, is a very slow remodeling rate in the cortical and trabecular bone. Remodeling is the turnover of bone tissue resulting from the linked processes of bone resorption and bone formation. Bone resorption is mediated by osteoclastic cells, which are derived from stem cell progenitors. Bone formation is thought to be mediated by osteoblastic cells, which are derived from mesenchyme cell progenitors. Both stem cells and mesenchyme cells are produced in bone marrow and may themselves have a common lineage. In the normal adult, these two processes are nearly equal, absolute bone loss is minimal and the remodeling (bone turnover) rate is six to nine months. The osteoporotic skeleton often requires two years or more for remodeling to occur. As a result of this slow remodeling process, microfractures in the reduced bone mass are only slowly repaired and occasionally progress to become major fractures. The primary complications of the disease are crush fractures of the vertebrae (which consist primarily of trabecular bone) and fractures of the neck of the femur and distal end of the radius (which consist of both cortical and trabecular bone).

Although the etiology of the disease is unknown, it is probably multifactorial. Empirical treatment is usually aimed at decreasing bone resorption and/or increasing the production of new bone, thereby lessening the net degeneration of skeletal integrity. Oral calcium supplements and sex hormones seem to decrease bone resorption. Administration of thyroxin, or feeding diets that are high in phosphorus and low in calcium for a period of time, seems to stimulate the bone-formation process. A number of other treatments have been tried. These treatments, the possible causes of osteoporosis and the various manifestations of the disease are discussed by Riggs et al., New Eng. J. Med. 314:1676 (1986), which is hereby incorporated by reference.

Another aspect of the invention relates to treating mammals for bone fractures, particularly those which fail to unite, where so-called "non-unions" occur. Stimulation of mesenchymal cells from around the fracture site is required in cases of fractures where non-unions occur. In non-unions, there does not appear to be a proper stimulation signal to the mesenchymal cells to start callus formation after a fracture occurs. The formation of callus with development of the trabeculae of woven bone that has an accompanying marrow component is an important aspect of the bone-healing process.

At present, the standard method of stimulating fracture non-union sites is surgery and either pinning or plating. Recently, non-union fractures have been treated with an experimental material called bone-morphogenic protein (BMP), which is placed surgically at the site of the non-union to stimulate local bone formation. Bone-morphogenic protein is a group of proteins existing in bone matrix and intimately associated with collagen fibrils. Isolation and characterization are difficult because BMP exists in its natural state in the hard bone matrix, but U.S.P. 4,761,471 (granted August 2, 1988) describes the isolation and substantial purification of bone morphogenic protein (which it terms 'Bone Morphogenetic Protein'). It has however been reported that solubilizing the matrix seems to alter the protein; Urist et al., J. Dent. Res. Suppl. No. 6, 50:1392 (1971); Urist et al., Cell Biology 76(4):1828 (1979).

Granulocyte macrophage colony stimulating factor (GM-CSF) is a lymphokine that stimulates the proliferation of a variety of undifferentiated precursor cells. Various cellular comoponents of the bone marrow are stimulated to proliferate by GM-CSF: MacDonald et al., J. Bone Mineral Res., 1(2):227 (1986); Begley et al., Exp. Hematol., 13:956 (1985).

### SUMMARY OF THE INVENTION

One aspect of this invention involves treating mammals diagnosed as having osteoporosis. The invention involves a method of treating such mammals by admin istering to them an effective anti-osteoporotic amount of a granulocyte macrophage colony stimulating factor (GM-CSF). Such an effective amount would reestablish and maintain bone-remodeling rates that are about normal.

Preferably, the mammals treated will be humans and the GM-CSF used will be one of the human allotypes. More preferably, administration of the human GM-CSF (hGM-CSF) will initially be daily to reestablish a normal bone-remodeling rate and later be periodical to maintain a normal bone-remodeling rate.

Preferably, the dosage for mammals will be about 1 to 300 $\mu$g of GM-CSF per kg of body weight per day. More preferably the dosage for humans will be about 2 to 10 $\mu$g of hGM-CSF per kg of body weight per day.

Another aspect of the invention involves administering an effective callus-forming amount of GM-CSF to stimulate mesenchymal stem cells around a fracture site to start callus formation, which is an important prerequisite to the bone-healing process.

Another aspect of the invention involves pharmaceutical combinations of GM-CSF and other stimulators of bone formation or inhibitors of bone resorption.

## Detailed Description

The invention provides a method for treating mammals diagnosed as having the disease osteoporosis by administering an effective anti-osteoporotic amount of a granulocyte macrophage colony stimulating factor (GM-CSF). Typically, the treatment will continue for a number of days and at a frequency necessary to maintain about normal bone-remodeling rate.

Any suitable GM-CSF may be employed in the present invention. Complementary DNAs (cDNAs) for GM-CSF have recently been cloned and sequenced by a number of laboratories: e.g. Gough et al., Nature 309:763 (1984) (mouse); Lee et al., Proc. Natl. Acad. Sci 82:4360 (1985) (human); Wong et al., Science 228:810 (1985) (human and gibbon); Cantrell et al., Proc. Natl. Acad. Sci, 82:6250 (1985) (human). Moreover, non-recombinant GM-CSF has been purified from various culture supernatants: e.g. U.S. Patent 4,438,032 (Mo cell line); Burgers et al., Exp. Hematol. 9:893 (1981) (mouse); Sparrow et al., Proc. Natl. Acad. Sci., 82:292 (1985) (purification and partial amino acid sequence for mouse); Wu et al., Exp. Hematol., 12:267 (1984) (rat); Gasson et al., Science 226:1339 (1984) (human); Sieff et al., Science 230:1171 (1985) (human); Burgess et al., Blood, 69:43 (1987) (human). Among the human GM-CSFs, heterogeneity in the sequences of the nucleotides and of the amino acids has been observed. For example, at the amino acid level of hGM-CSF both threonine and isoleucine have been observed at position 100 with respect to the N-terminal alanine, suggesting that allelic forms, or polymorphs, of GM-CSF may exist within human populations. Also, various leader sequences occur at the N-terminal portion of the amino acid sequence. These leaders may be of various lengths and amino acid composition, which may or may not affect biological activity. All of the above discussed references are incorporated herein by reference for their disclosures of representative GM-CSFs suitable for use in the present invention.

According to this invention, mammals with osteoporosis are administered an effective anti-osteoporotic amount of a GM-CSF. Preferably, an amount is used sufficient to reestablish bone-remodeling rates to about normal and maintain them at about normal. From about 1 to about 300 $\mu$g of human GM-CSF (hGM-CSF) per kg of body weight per day is preferably administered. More preferably, osteoporotic mammals (e.g. humans) are administered 1 to 100 $\mu$g of GM-CSF (e.g. hGM-CSF) per kg of body weight per day. Most preferably, osteoporotic humans are administered 2 to 10 $\mu$g of hGM-CSF per kg of body weight per day.

The amount, frequency and period of administration will vary depending upon factors such as severity of the disease, age of the patient, activity of the patient, nutrition, etc. Usually, the administration will initially be daily and later be less frequent, and it may continue periodically during the remainder of the patient's lifetime. Dosage amount and frequency may be determined during initial screenings of bone-remodeling rate and the magnitude of the effect of GM-CSF upon the remodeling rate for specific patients. Dosage will be aimed at returning remodeling rate to as near normal as possible.

Administration of the dose may be intravenous, nasal, parental, oral, subcutaneous, intramuscular, topical, transdermal or by any other accepted method. Such administration may be by pulse dosing, such as administration for 3 to 7 days followed by periods of rest without further drug administration, or further ('second') dosing etc.

Since bone reformation is a sequential mechanism with multifactoral regulation, it is likely that the positive effect of GM-CSFs on bone remodeling can be enhanced when these are used in combination with other compounds. Such stimulators of bone remodeling include bone-formation stimulators and bone-resorption inhibitors. This invention encompasses such pharmaceutical combinations. Bone formation can be stimulated, for example, by administration of sodium fluoride, parathyroid hormone, prostaglandins, thyroxin, bone-morphogenic protein and high-phosphate diet. Bone resorption can be inhibited, for example, by administration of diphosphonates, calcitonin, glucocorticoids and high-calcium diet. The availability, source, activity, effect and use of these and other bone-formation stimulators and bone-resorption inhibitors are well known to those skilled in the art. In the present invention, their use will be in pharmaceutical combination with the GM-CSF or in combination with a compatible GM-CSF as part of the dosage regimen. These stimulators and inhibitors may or may not be combined with every administration of GM-CSF. The timing and amount of administration will be determined by the effect of specific stimulators and inhibitors on the events occurring during bone reformation.

The effect of GM-CSF on remodeling rates can be determined by the following test protocol, which is well known to those skilled in the art as a method of determining the effects of a given dosage regimen for a drug on increasing bone-remodeling rates:

Patients are dosed with tetracycline one to three weeks prior to treatment with the GM-CSF. Tetracycline may be administered in a single dose or in several repeated doses. GM-CSF is administered at a defined dosage level and regimen for several weeks or months. This is followed by administration of another bone-labeling compound, DCAF. DCAF is a fluorochrome label [(2,4 bis)N,N'-di(carboxymethyl)-(aminomethylfluoroescein)], which can be obtained from ICN Pharmaceuticals, Inc.

Tetracycline, which fluoresces green, and DCAF, which fluoresces red, are both incorporated into newly forming bone-fronts. Therefore, if tetracycline is administered to a mammal with a fracture, and DCAF is later administered, an examination of a section of the healing fracture will provide information about the bone-remodeling rate. If parallel experiments are done upon different mammals, some of which are administered GM-CSF, and some of which receive no GM-CSF, then the said measurement and comparison will provide information about the relative bone-remodeling rates in the presence of GM-CSF and in its absence.

Fluorescence is determined in bone cross-sections obtained from bone biopsies conducted before treatment with GM-CSF and after treatment with GM-CSF and DCAF. Entire rib cross-sections are obtained in biopsies conducted in monkeys, whereas iliac crest biopsy is the preferred method for obtaining bone cross-sections in humans.

Once the bone cross-sections are obtained, the following nine measurements and counts are obtained for cortical bone:

(1) Cortical area ($A_C$)
(2) Total area ($A_T$)
(3) Total number of osteoid seams ($A_f$)

The mean number was expressed per $mm^2$ of cortical area ($A_C$). An osteoid seam was identified as a bone formation site by a green or red intimal band on sections stained with osteochrome.

(4) Total number of resorption cavities ($A_r$)

Measured by systematic scanning of the entire section using a 10 X objective. Resorption cavities were identified by the presence of scalloped edges.

(5) Total number of labelled osteoid seams (L)
(6) Mean distance between fluorochrome dyes within a single Haversian system (MAR)
(7) Mean wall thickness of finished osteons (MWT)
(8) Osteoid seam thickness (OST)
(9) Mean circumference of osteoid seams ($S_f$)

From the nine basic measurements, the quantitative morphometric analysis of bone consists of the following stereological calculations disclosed by Frost, Bone Remodeling and Its Relationship to Metabolic Bone Diseases, Orthopedic Lectures, Vol. III, Charles Thomas Publisher, (1973), which is hereby incorporated by reference:

(1) Total Cortical Cross Sectional Area ($A_C$) in $mm^2$
(2) Total Cross Sectional Area ($A_T$) in $mm^2$

$$A_C \text{ or } A_T = \frac{\text{No. of hits}}{(\text{grid factor})(\text{No. of throws})(\text{No. of intersections})}$$

("Hits" and "throws" are well-understood terms of a scoring system used to determine cross-section on a microscopic grid; they are also described in the Frost reference immediately above.)

(3) Mean Number of Osteoid Seams per mm² ($A_f$)

(4) Mean Number of Resorption Cavities per mm² ($A_r$)

$$A_f \text{ or } A_r = \frac{\text{Total No. of Osteoid Seams of Resorption Cavities}}{\text{Total Cortical Area } (A_C) \text{ mm}^2}$$

(5) Percent labelled Osteoid Seams (%L)

$$\%L = \frac{100(\text{Total No. of Labelled Osteoid Seams})}{\text{Total No. of Osteoid Seams}}$$

(6) Mean Appositional Rate (M) in $\mu$/day

$$M = \frac{\text{Mean Distance Between Two Labels (MAR) in microns}}{\text{No. of Days given second Label}}$$

(7) Radial Closure Rate ($M_f$) in mm/yr.

$M_f = (M) \times (\%L) \times (365/1000^*)$

*reduces microns/day to mm/year

(8) Osteoid Seam Circumference ($S_f$)

$$S_f = \frac{(\text{No. of Hits})(\text{Distance Between Parallel Grid}) \ (\pi)}{(2) \ (\text{No. of Throws})}$$

(9) Ratio of Cortical (C) Area in mm² ($A_c$) to Total (T) Area in mm² ($A_T$)

$$C/T = \frac{A_C}{A_T}$$

(10) Ratio of the Numbers of Resorption Cavities ($A_r$) to Osteoid Seams ($A_f$)

$A_r/A_f$

(11) Osteon Formation time in Years ($O_f$)

The time required to complete lamellar bone formation in a cross section of an average osteon.

$$O_f = \frac{MWT}{M_f}$$

(12) Number of Activation Frequency Foci in mm²/year ($\mu_f$)

$$\mu_f = \frac{A_f}{O_f}$$

$\mu_f$ represents the, number of new osteons introduced per year in an average mm² of cortical area.

(13) Bone Formation Rate in mm² of new bone per mm² of existing bone surface per year ($V_f$)

$$V_f = (A_f) \times (S_f) \times (M_F)$$

Statistical significance of differences in the morphometric data comparing treatment effects between GM-CSF-dosed bone samples and control bone samples are determined by the Nemenyl Rank Sum One-Way Classification test of Dixon et al., Biomedical Computer Programs: P-Series, BMDT 77:603-652 (University of California-Berkeley Press) (1977), which is hereby incorporated by reference.

The invention also provides a method for treating mammals with fractures by stimulating mesenchymal stem cells with GM-CSF. GM-CSF is used to activate the mesenchymal cells around the fracture site and thereby stimulate them to produce callus. Callus formation leads to development of trabeculae and accompanying bone marrow.

The GM-CSF is administered systemically or locally in sufficient amounts to induce the formation of callus. Systemic and local administration are methods known to one skilled in the art. Systemic administration indicates any means of introducing the pharmaceutical composition into the blood stream, including intraveneous, parental, subcutaneous, intramuscular, oral, nasal, topical and transdermal. In local administration, the pharmaceutical composition is applied directly to the vicinity of the cells to be affected, which here are the cells adjacent to the fracture area. Such administration includes injections or surgically applied dosages. One successful application of bone morphogenic protein is to surgically apply a capsule or other slowly-dissolving or sustained-released dosage directly to the fracture area. The amount, frequency and method of administration will depend upon the extent of non-union in the fracture. Preferably, the GM-CSF is human GM-CSF.

As with the treatment of osteoporosis with GM-CSF, the positive effect of GM-CSFs on bone reformation can be enhanced when used in combination with other compounds. Such stimulators of bone reformation include the same bone-formation stimulators and bone-resorption inhibitors as are discussed above. Preferably, the bone-formation stimulators, including sodium fluoride, parathyroid hormone, prostaglandins, thyroxin, bone-morphogenic protein, local electrical stimulation and high-phosphate diet, are administered. In this aspect of the invention, their use will be in pharmaceutical combination with the GM-CSF or in combination with the GM-CSF as part of the dosage regimen. These stimulators and/or inhibitors may or may not be combined with every administration of GM-CSF.

The effect of GM-CSF on bone formation can be determined by the following test protocols, which are well known to those skilled in the art as a method of determining the effect of a given dosage regimen of a drug on increasing callus formation:

The effect of GM-CSF on bone formation is determined by studying the fracture-healing process over time. The initial effect of GM-CSF upon the stimulation of mesenchymal cells around the new fracture site is determined by the incorporation of tritiated thymidine into nuclei of newly generated cells at the fracture site. This technique, applied over the first week of the study, uses autoradiography as the method to determine the rate of generation of new cells, as stimulated by GM-CSF. Further study of the fracture-healing process uses the sequential microscopic evaluation of callus formation using undercalcified tissue sections collected from animals sacrificed at selected intervals after fracture. The sections are stained for fibrous stroma or mineralized callus. The amount of fibrous stroma as well as the mineralized component of the callus is quantitated using quantitative image analysis. In addition, polarized light microscopy is used to evaluate the morphologic character of the callus and to detect the presence or absence of union across the opposing edges of the shaft of the bone. After approximately two weeks the fracture-healing process is further followed by the use of sequential x-rays on live animals. Finally, the quality of the callus after approximately six weeks is determined by the application of measured mechanical forces to the fracture site in both tension and flexure. The breaking strength is thereby determined.

6

## Claims

1. A method for treating a mammal diagnosed as having osteoporosis comprising administering to said mammal an effective anti-osteoporotic amount of a granulocyte macrophage colony stimulating factor (GM-CSF).

2. The method of treatment of claim 1 wherein said GM-CSF is administered in an amount of about 1 to about 300 μg per kg of body weight per dose.

3. The method of treatment of claim 1 wherein said GM-CSF is human GM-CSF.

4. The method of treatment of claim 3 wherein said human GM-CSF is administered in an amount of about 1 to about 10 μg per kg of body weight per dose.

5. The method of treatment of claim 4 wherein the mode of administration is selected from the group consisting of intravenous, parental, subcutaneous, intramuscular, oral, nasal, topical and transdermal.

6. The method of treatment of claim 4 wherein the mode of administration is nasal or oral.

7. The method of treatment of claim 4 wherein the mode of administration is topical or transdermal.

8. The method of treatment of claim 4 wherein the mode of administration is intravenous, parental, subcutaneous or intramuscular.

9. The method of treatment of claim 4 wherein said dose is administered about daily until the bone-remodeling rate is about normal and at a frequency thereafter to maintain about a normal bone-remodeling rate.

10. The method of treatment of claim 1 further comprising administration of GM-CSF in combination with one or more stimulators of the bone-reformation process in an effective amount to reestablish and maintain bone-remodeling rates at about normal.

11. The method of treatment of claim 10 wherein said GM-CSF is human GM-CSF.

12. The method of treatment of claim 11 wherein said stimulators of the bone-reformation process are selected from the group consisting of bone-formation stimulators and bone-resorption inhibitors.

13. The method of treatment of claim 12 wherein said bone-formation stimulators are selected from the group consisting of sodium fluoride, parathyroid hormone, prostaglandins, thyroxin, bone-morphogenic protein and high phosphate diet.

14. The method of treatment of claim 13 wherein said bone-resorption inhibitors are selected from the group consisting of diphosphonates, calcitonin, glucocorticoids and high-calcium diet.

15. A method for treating mammalian bone fractures comprising administering to said mammal an effective callus-forming amount of a granulocyte macrophage colony stimulating factor (GM-CSF) to stimulate callus formation.

16. The method of treatment of claim 15 wherein the mode of administration is systemic.

17. The method of treatment of claim 15 wherein the mode of administration is local.

18. The method of treatment of claim 15 further comprising administering GM-CSF in combination with one or more stimulators of the bone-reformation process in an effective amount to stimulate production of callus.

19. A pharmaceutical composition comprising an effective amount of GM-CSF, said amount selected from an effective anti-osteoporotic amount and an effective callus-forming amount, in combination with one or more stimulators of the bone-reformation process.

20. The pharmaceutical composition of claim 19 wherein said GM-CSF is human GM-CSF.

21. The pharmaceutical composition of claim 20 wherein said stimulators of the bone-reformation process are selected from the group consisting of bone-formation stimulators and bone-resorption inhibitors.

22. The pharmaceutical composition of claim 21 wherein said bone-formation stimulators are selected from the group consisting of sodium fluoride, parathyroid hormone, prostaglandins, thyroxin, bone-morphogenic protein, local electrical stimulation and high-phosphate diet.

23. The pharmaceutical composition of claim 21 wherein said bone-resorption inhibitors are selected from the group consisting of diphosphonates, calcitonin, glucocorticoids and high-calcium diet.

European Patent Office

**PARTIAL EUROPEAN SEARCH REPORT**
which under Rule 45 of the European Patent Convention
shall be considered, for the purposes of subsequent
proceedings, as the European search report

Application number

EP 88 31 0594

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,Y | BIOLOGICAL ABSTRACTS/RRM, no. 3508506 D.B. EVANS et al.: "Granulocyte-macrophage colony-stimulating factor GM-CSF exhibits regulatory actions on human osteoblast-like obs cells in-vitro" & 2nd INTERNATIONAL WORKSHOP ON CELLS AND CYTOKINES IN BONE AND CARTILAGWE, Davos, CH, April 9-12, 1988, Calcif tissue Int. 1988, vol. 42, no. suppl. pA17, * Title and terms * | 19-23 | A 61 K 37/36 A 61 K 37/24 |
| Y | EP-A-0 197 514 (THE GENERAL HOSPITAL CORP.) * Page 6, 2nd paragraph; page 7, paragraphs 1,2; page 11, paragraph 1; page 13, paragraph 1; claims 1,3 * | 19-23 | |
| | ./. | | **TECHNICAL FIELDS SEARCHED (Int. Cl.4)**<br><br>A 61 K<br>C 12 P |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 19-23

Claims searched incompletely:

Claims not searched: 1-18

Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 03-03-1989 | CHARLES |

| Category | Citation of document with indication, where appropriate, of relevant passages | Relevant to claim |
|---|---|---|
| A | FR-A- 893 138 (G. GLAESSNER)<br><br>* Page 1, lines 1-38; claim 1 *<br><br>-- | 19-23 |
| A | CHEMICAL ABSTRACTS, vol. 93, no. 5, August 4, 1980, page 165, abstract no. 37924a; Columbus, Ohio, US; N. WILLIAMS et al.: "The effect of mouse lung granulocyte-macrophage colony-stimulating factor and other colony-stimulating activities on the proliferation and differentiation of murine bone marrow cells in long-term cultures" & J. CELL. PHYSIOL. 1980, 102(3), 287-95<br><br>* Abstract *<br><br>------- | 19-23 |

## DOCUMENTS CONSIDERED TO BE RELEVANT

CLASSIFICATION OF THE APPLICATION (Int. Cl.4)

TECHNICAL FIELDS SEARCHED (Int Cl.4)

EPO Form 1505.3  06.78